# EUROPEAN PATENT SPECIFICATION

(11) **EP 2 875 838 B1**
(45) Date of publication and mention of the grant of the patent: **19.04.2017**
(21) Application number: 12881488.6
(22) Date of filing: 19.07.2012
(51) Int. Cl.: A61M 5/315, A61M 5/50, A61M 5/32, A61M 5/28

(54) **LIQUID-ADMINISTERING INSTRUMENT**
FLÜSSIGKEITSVERABREICHENDES INSTRUMENT
INSTRUMENT D'ADMINISTRATION DE LIQUIDE

(43) Date of publication of application: 27.05.2015
(73) Proprietor: Terumo Kabushiki Kaisha, Tokyo 151-0072 (JP)
(72) Inventor: FUKE, Shigeaki, Ashigarakami-gun Kanagawa 259-0151 (JP); IMAI, Masaomi, Ashigarakami-gun Kanagawa 259-0151 (JP); ARINOBE, Manabu, Ashigarakami-gun Kanagawa 259-0151 (JP)
(74) Representative: Casalonga
(86) International application number: PCT/JP2012/068367
(87) International publication number: WO 2014/013594

(56) References cited:
- WO-A1-2009/013844
- WO-A1-2009/040602
- JP-A- 2004 049 726
- JP-A- 2009 095 392

## Description

### Technical Field

The present invention relates to a liquid administration device, and in particular to a liquid administration device according to the preamble of claim 1, such as it is for example known from WO 2009/040602 A1.

### Background Art

There has also been known a prefilled syringe, which is filled with a liquid preparation in an aseptic manner and capable of administering the liquid preparation (see Patent Literature 1). The prefilled syringe disclosed in Patent Literature 1 includes a syringe outer cylinder, a gasket, the liquid preparation, and a plunger. The syringe outer cylinder has an opening from which the liquid preparation discharges. The gasket can slide within the syringe outer cylinder. The space surrounded by the syringe outer cylinder and the gasket is filled with the liquid preparation. The plunger is coupled to the base end side of the gasket and discharges the liquid preparation from the opening by pressing the gasket toward the tip direction. In this prefilled syringe, the opening of the syringe outer cylinder is sealed liquid-tightly with a cap in an unused state. When the liquid preparation is administered using the prefilled syringe, first, the cap is removed from the opening of the syringe outer cylinder, and then, an injection needle is mounted on the opening from which the cap is removed. Next, a living body is punctured with the injection needle, and pressing operation by the plunger is performed in this punctured state. As a result, the liquid preparation is discharged from the opening by the gasket, thus, the liquid preparation can be administered to the living body though the injection needle.

However, generally the pressing operation of the plunger of the prefilled syringe can be performed in an arbitrary timing of a user once the cap is removed. Therefore, there may have been a problem, in which the pressing operation of the plunger is performed erroneously before the living body is punctured with the injection needle. In this case, the liquid preparation leaks from the injection needle unintentionally, or it has been sometimes difficult to administer a sufficient amount of the liquid preparation to the living body since the liquid preparation is insufficient due to the leakage.

Patent Literature 1: JP 2005-319118 A

### Summary of Invention

### Technical Problem

An object of the present invention is to provide a liquid administration device which can surely prevent an operation member from operating erroneously.

### Solution to Problem

Such an object is achieved by (1) to (7) described below in the present invention.

(1) A liquid administration device including:
   a cylindrically-shaped cylindrical body configured to be filled with liquid in its inside;
   a needle tube configured to be provided being communicated or communicable with the cylindrical body at a tip side of the cylindrical body, and to have a sharp needle tip at a tip;
   a gasket configured to be capable of sliding within the cylindrical body;
   an operation member configured to perform pressing operation to discharge the liquid from the needle tube by pressing the gasket toward a tip direction;
   a restricting member configured to be arranged at a outer peripheral side of the cylindrical body, and to be capable of restricting the pressing operation; and
   a cover member configured to be capable of moving to a first position, in which at least the needle tip of the needle tube is covered, and to a second position, retreated to a base end direction from the first position, in which the needle tip is exposed, wherein
   a slider part is provided to one member out of the operation member and the restricting member, and a rail part, in which the slider part slides, is provided to the other member, the rail part having a longitudinal direction rail formed in the same direction as an operation direction during the pressing operation, and a lateral direction rail continued to the longitudinal direction rail and formed in the direction perpendicular to the operation direction;
   when the cover member is in the first position, the slider part is positioned in the lateral direction rail and makes the pressing operation impossible, and
   when the cover member moves from the first position to the second position, the slider part moves from the lateral direction rail to the longitudinal direction rail in conjunction with the movement, thereby the pressing operation is made possible.
(2) The liquid administration device described in the above (1), wherein
   the restricting member is cylindrically-shaped;
   the operation member has a column-shaped plunger arranged concentrically with the restricting member inside the restricting member; and
   the slider part is provided in an inner peripheral part of the restricting member along its circumferential direction, and the rail part is arranged so as to correspond to each of the slider parts in an outer peripheral part of the plunger.
(3) The liquid administration device described in the above (1) or (2), wherein
   the restricting member is cylindrically-shaped;
   the cover member is arranged concentrically with the restricting member outside the restricting member and is cylindrically-shaped;
   an outer slider part, positioned outside compared with the slider part, is provided in one of the outer peripheral part of the restricting member and the inner peripheral part of the cover member, and an outer rail part, in which the outer slider part slides, is provided in the other, the outer rail part having an outer inclined rail formed in the direction inclined relative to the operation direction; and
   in the process in which the cover member moves from the first position to the second position, the outer slider part slides in the outer inclined rail, such that the restricting member rotates around its axis and the slider part slides from the lateral direction rail to the longitudinal direction rail, thereby the pressing operation is made possible.
(4) The liquid administration device described in any one of the above (1) to (3), wherein
   the cover member can further move to a third position, in which the needle tip is covered and the movement to the second position is prevented; and
   the restricting member is configured to allow the movement of the cover member from the second position to the third position in conjunction with completion of the pressing operation.
(5) The liquid administration device described in the above (4), wherein
   the outer rail part has a first outer longitudinal direction rail continued to the base end of the outer inclined rail and formed in the same direction as the operation direction during the pressing operation, an outer lateral direction rail continued to a tip of the outer inclined rail and formed in the direction perpendicular to the operation direction, and a second outer longitudinal direction rail continued to the outer lateral direction rail and formed in the opposite direction to the operation direction during the pressing operation; and
   the outer slider part slides in the first outer longitudinal direction rail and the outer inclined rail in order in the process in which the cover member moves from the first position to the second position, the outer slider part slides in the outer lateral direction rail when the cover member is in the second position, and the outer slider part moves in the second outer longitudinal direction rail when the cover member moves from the second position to the third position.
(6) The liquid administration device described in the above (5), wherein
   the outer rail part has an engagement part, which restricts the cover member to move back to the second position by engaging with the outer slider part from its tip side when the cover member moves to the third position.
(7) The liquid administration device described in the above (6), wherein
   the engagement part is deformed elastically and
   allows movement of the cover member from the second position to the third position.

### Advantageous Effects of Invention

According to the present invention, it is possible to surely switch enabling/disabling of pressing operation by the operation member, according to the position of the cover member. Therefore, the operation member can be surely prevented from operating erroneously when the operation of the operation member is desired to be restricted.

In addition, since the movement of the operation member is surely restricted until the cover member is moved from a first position to a second position, increase in inner pressure of the liquid within the cylindrical body is prevented during this time.

### Brief Description of Drawings

Fig. 1 is a side view (figure in which a part has been cut) showing an operation state in use of a liquid administration device of the present invention in order.
Fig. 2 is a side view (figure in which a part has been cut) showing the operation state in use of the liquid administration device of the present invention in order.
Fig. 3 is a side view (figure in which a part has been cut) showing the operation state in use of the liquid administration device of the present invention in order.
Fig. 4 is a side view (figure in which a part has been cut) showing the operation state in use of the liquid administration device of the present invention in order.
Fig. 5 is a side view (figure in which a part has been cut) showing the operation state in use of the liquid administration device of the present invention in order.
Fig. 6 is a side view (figure in which a part has been cut) showing the operation state in use of the liquid administration device of the present invention in order.
Fig. 7 is a side view (figure in which a part has been cut) showing the operation state in use of the liquid administration device of the present invention in order.
Fig. 8 is a longitudinal sectional view of the liquid administration device in the state shown in Fig. 1.
Fig. 9 is a longitudinal sectional view of the liquid administration device in the state shown in Fig. 2.
Fig. 10 is a longitudinal sectional view of the liquid administration device in the state shown in Fig. 3.
Fig. 11 is a longitudinal sectional view of the liquid administration device in the state shown in Fig. 4.
Fig. 12 is a longitudinal sectional view of the liquid administration device in the state shown in Fig. 5.
Fig. 13 is a longitudinal sectional view of the liquid administration device in the state shown in Fig. 6.
Fig. 14 is a longitudinal sectional view of the liquid administration device in the state shown in Fig. 7.
Fig. 15 is an exploded perspective view showing a positional relation between an operation member and a restricting member included in the liquid administration device of the present invention.
Fig. 16 is an exploded perspective view showing a positional relation between the restricting member and a cover member included in the liquid administration device of the present invention.
Fig. 17 is view of the restricting member included in the liquid administration device of the present invention, seen from the tip side.
Fig. 18 is a perspective view showing a puncture needle included in the liquid administration device of the present invention.

### Description of Embodiment

Hereinafter, a liquid administration device of the present invention will be described in detail based on the preferred embodiment shown in the attached drawings.

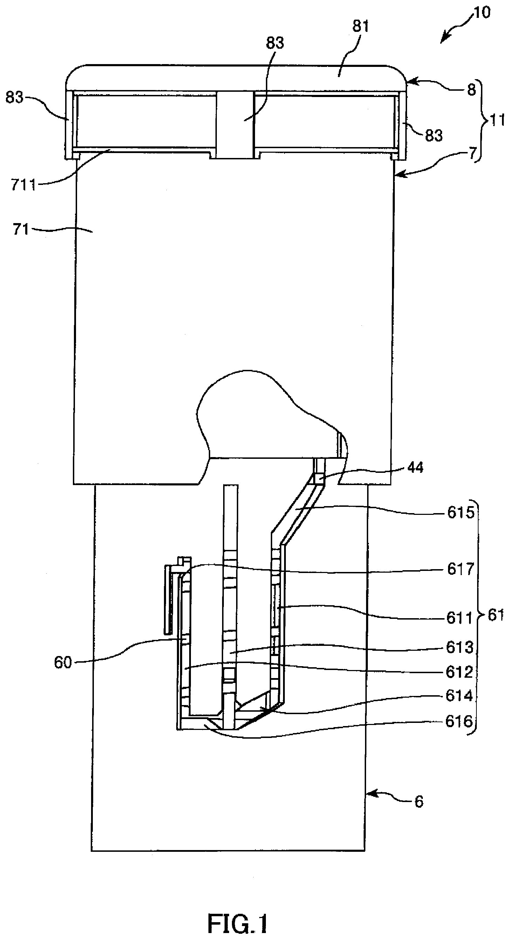

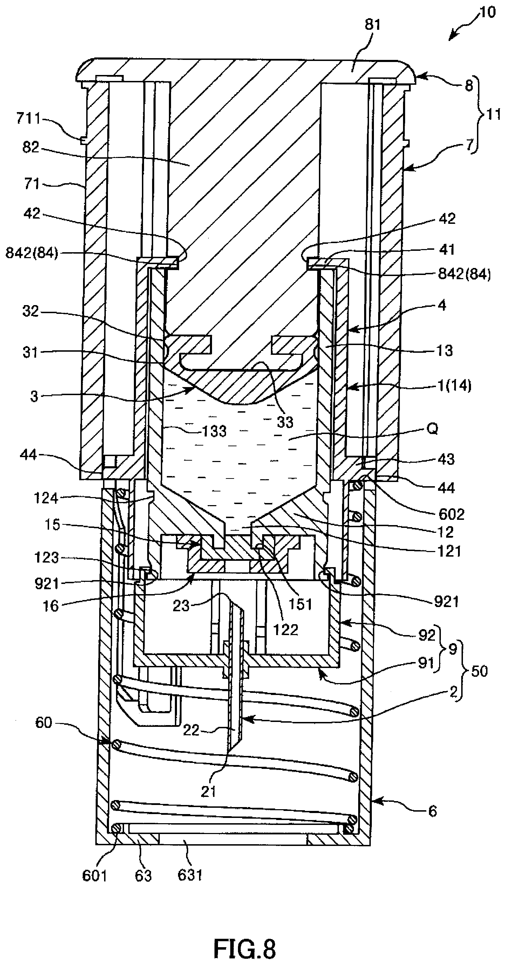

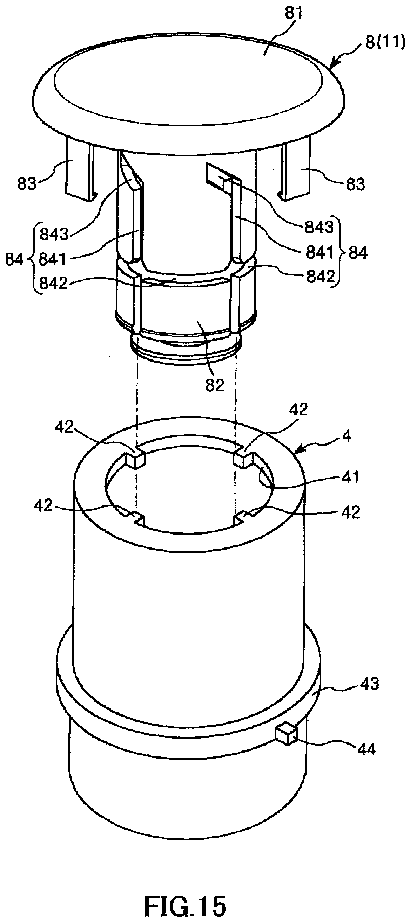

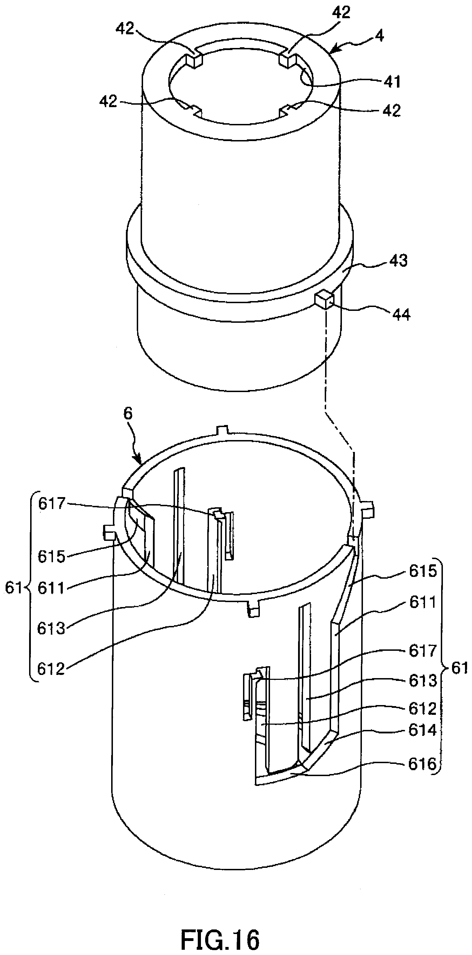

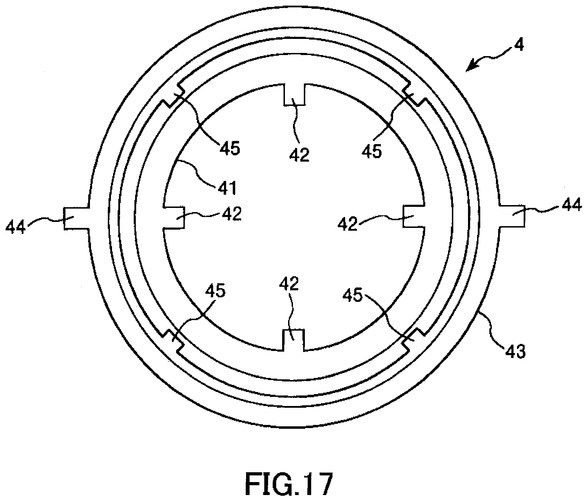

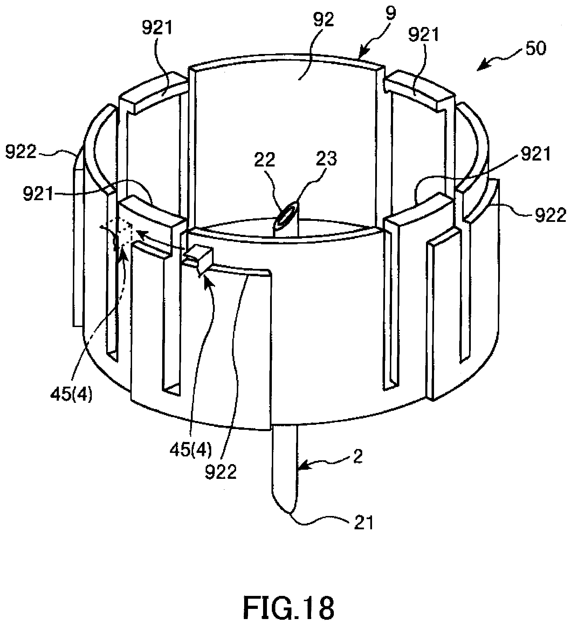

Each of Figs. 1 to 7 is a side view (figure in which a part has been cut) showing an operation state in use of the liquid administration device of the present invention in order. Each of Figs. 8 to 14 is a longitudinal sectional view of the liquid administration device in the state shown in Figs. 1 to 7. Fig. 15 is an exploded perspective view showing a positional relation between an operation member and a restricting member included in the liquid administration device of the present invention. Fig. 16 is an exploded perspective view showing a positional relation between the restricting member and a cover member included in the liquid administration device of the present invention. Fig. 17 is view of the restricting member included in the liquid administration device of the present invention, seen from the tip side. Fig. 18 is a perspective view showing a puncture needle included in the liquid administration device of the present invention. Hereinafter, for convenience of explanation, the upper side is referred to as "base end" or "upper (upper side)" and the lower side is referred to as "tip" or "lower (lower side) in Figs 1 to 16 and 18.

The liquid administration device 10 shown in Figs. 1 to 14 is a medical device used when liquid Q is administered (injected) into a living body B. The liquid Q is suitably selected according to its purpose of use. As the liquid Q, for example, medicinal liquids, mainly injected hypodermically, such as hematopoietic agent, vaccines, hormone preparations, antirheumatics, carcinostatic agents, anesthetics, and anticoagulants are raised.

The liquid administration device 10 includes an inner cylinder (cylindrical body) 1, a puncture needle 50, a gasket 3, an operation member 11, a restricting member 4, a cover member 6, and a coil spring 60 as a biasing means. The puncture needle 50 includes a double ended needle 2 and a support member 9. The gasket 3 can slide within the inner cylinder 1. The operation member 11 is coupled to the base end side of the gasket 3. The restricting member 4 restricts operation of the operation member 11. The cover member 6 is arranged at the outer peripheral side of the inner cylinder 1. The coil spring 60 biases the cover member 6.

As shown in Figs. 8 to 14, the inner cylinder 1 has an inner cylinder body 14, which is a member including a bottom part 12 at the tip and a side wall 13 erected from the edge of the bottom part 12, that is, a member with a bottomed cylindrical shape. Then, inside of the inner cylinder 1 can be filled with the liquid Q.

The bottom part 12 has a mortar shape. An opening part 121, through which the liquid passes, is formed at center of the bottom part 12.

The side wall 13 has a cylindrical shape.

In addition, the inner cylinder 1 has a sealing member (sealing part) 15, which seals the opening part 121 of the inner cylinder body 14 liquid-tightly, and a fixing member 16, which fixes the sealing member 15 from its tip side.

The sealing member 15 is a disk-shaped elastic piece, and a ring-shaped recessed part 151 is formed on its base end surface. A ring-shaped protruded part 122, which is formed protrudedly on the opening part 121 of the inner cylinder body 14, can fit liquid-tightly to this recessed part 151. As a result, the sealing member 15 is attached to the opening part 121 of the inner cylinder body 14 and can seal the opening part 121 liquid-tightly.

The fixing member 16 is a ring-shaped member. Then, this fixing member 16 fits to the sealing member 15 from its outer peripheral side, and holds the sealing member 15 between the fixing member 16 and the protruded part 122. Thus, the sealing member 15 can be surely fixed to the inner cylinder body 14. As a result, removal of the sealing member 15 from the inner cylinder body 14 is surely prevented. The method by bonding or welding may also be used as a method to fix the fixing member 16.

In addition, a constituent material for the inner cylinder body 14, the fixing member 16, the cover member 6, the support member 9, the operation member 11, and the restricting member 4 are not particularly limited. Examples include various types of resins such as polyvinyl chloride, polyethylene, polypropylene, cyclic polyolefins, polystyrene, poly(4-methylpentene-1), polycarbonates, acrylic resins, acryl nitrile-butadiene-styrene copolymer, polyesters such as polyethylene terephthalate and polyethylene naphthalate, butadiene-styrene copolymer, and polyamides (e.g., nylon 6, nylon 6-6, nylon 6-10 and nylon 12). Of these, it is preferred to use resins such as polypropylene, cyclic polyolefins, polyesters and poly-(4-methylpentene-1) in view of the ease in molding.

In addition, an elastic material included in the sealing member 15 and the gasket 3 is not particularly limited. Examples include various rubber materials such as natural rubber, butyl rubber, isoprene rubber, butadiene rubber, styrene-butadiene rubber, silicone rubber, various types of thermoplastic elastomers, such as polyurethane, polyester, polyamide, olefin, and styrene elastomers, or elastic materials such as mixtures thereof.

The puncture needle 50 is arranged at the tip side of the inner cylinder 1. The puncture needle 50 includes the double ended needle 2 and the support member 9.

The double ended needle 2 is a hollow needle tube, which has a sharp tip side needle tip (needle tip) 21 at the tip and a sharp base end side needle tip 23 also at the base end. In this double ended needle 2, the living body B can be punctured with the tip side needle tip 21 and the sealing member 15 of the inner cylinder 1 can be pierced with the base end side needle tip 23.

A lumen part (hollow part) of the double ended needle 2 is communicating with the inner cylinder 1, in a state where the base end side needle tip 23 is piercing the sealing member 15 of the inner cylinder 1, and functions as a flow path 22, through which the liquid Q from the inner cylinder 1 passes. Then, the liquid Q is injected into the living body B through the flow path 22, in a state where the living body B is punctured to a predetermined depth from the skin with the tip side needle tip 21.

A constituent material for the double ended needle 2 is not particularly limited, and for example, metallic materials such as stainless steel, aluminum or aluminum alloys, and titanium or titanium alloys are raised.

The double ended needle 2 with such a configuration is attached to the inner cylinder 1 through the support member 9. The support member 9 movably supports the double ended needle 2 relative to the inner cylinder 1 along its center axis direction. This support member 9 includes a disk-shaped fixing part (support part) 91 and a wall part 92 erected toward the base end direction from the edge of the fixing part 91.

The fixing part 91 can support and fix the double ended needle 2 at its center. The part supported by the fixing part 91 of the double ended needle 2 is in midway in the longitudinal direction of the double ended needle 2.

The wall part 92 is a ring-shaped part along the edge of the fixing part 91. As shown in Fig. 18, a plurality (four in the configuration shown in Fig. 8) of engagement parts 921, which protrudes to inside, is formed at the inner peripheral part of the base end of the wall part 92. Each of engagement parts 921 is arranged at equal intervals along the circumferential direction of the wall part 92. Each of engagement parts 921 can be engaged with a first engagement part 123 or a second engagement part 124, which is formed recessedly at the edge of the bottom part 12 of the inner cylinder 1, according to the position of the puncture needle 50 relative to the inner cylinder 1 (see Figs. 8, 9, and 11 to 14). Then, removal of the puncture needle 50 from the tip of the inner cylinder 1 is prevented, in either of a state where each of engagement parts 921 is engaged with the first engagement part 123 and a state where each of engagement parts 921 is engaged with each of engagement parts 921.

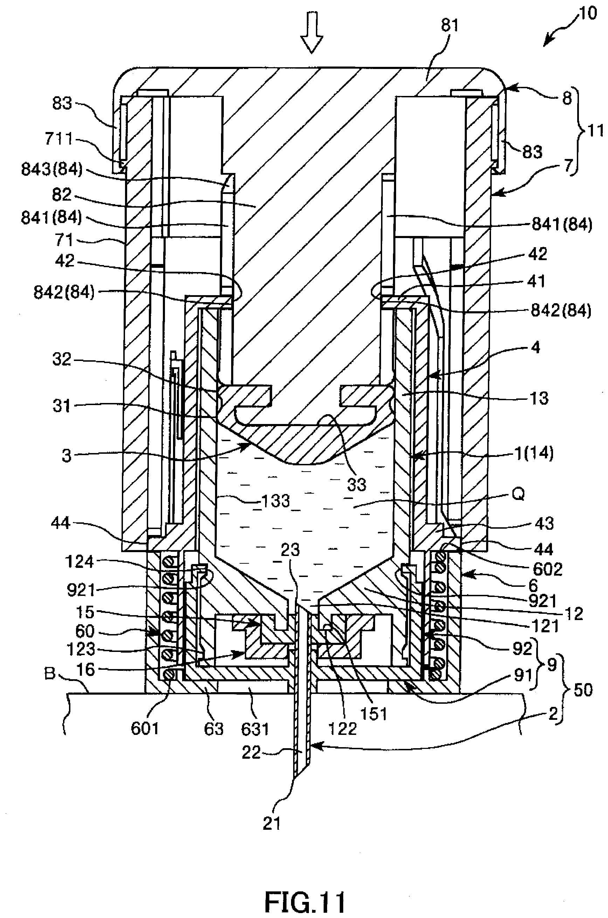

As described above, the puncture needle 50 is movably supported relative to the inner cylinder 1 along the center axis direction through the support member 9. As a result, the puncture needle 50 can take a state where the base end side needle tip 23 is separated from (or not pierced) the sealing member 15 of the inner cylinder 1 as shown in Figs. 8 to 10, and a state where the base end side needle tip 23 pierces the sealing member 15 as shown in Figs. 11 to 14. Therefore, unintentional leaking of the liquid Q from the double ended needle 2 is prevented until a pierced state is realized.

In the separated state, each of engagement parts 921 is engaged with the first engagement part 123, and in the pierced state, each of engagement parts 921 is engaged with each of engagement parts 921.

The gasket 3 is slidably stored along the axis direction of the inner cylinder 1. The space, surrounded by this gasket 3 and the inner cylinder 1, is preliminarily filled with the liquid Q. The gasket 3 moves toward the tip direction, so that the liquid Q in the inner cylinder 1 can be pushed out from the double ended needle 2, which is communicating with the inner cylinder 1.

An outer shape of this gasket 3 is disk-shaped, and two protruded parts 31 and 32 are protrudedly formed at its outer peripheral part. The protruded parts 31 and 32 are separated along the axis direction of the gasket 3. Each of protruded parts 31 and 32 is ring-shaped along the circumferential direction of the gasket 3, and the outer diameter thereof, in a natural state where external force is not given, is slightly larger than the inner diameter of the inner cylinder 1. As a result, each of protruded parts 31 and 32 can slide while closely in contact with an inner peripheral part 133 of the side wall 13 of the inner cylinder 1. Thus, it is possible to surely retain the liquid-tightness and improve slidability.

At a base end surface of the gasket 3, in which the recessed part 33 to be coupled is opened, the tip of a plunger 82 of the operation member 11 is inserted (fitted).

The operation member 11 is a member to perform the pressing operation (discharging operation) to discharge the liquid Q from the double ended needle 2 by pressing the gasket 3 toward the tip direction. The operation member 11 has an operation member body 8 to be coupled to the gasket 3, and a holding member (holding part) 7 to be held when the pressing operation is performed.

As shown in Fig. 15, the operation member body 8 has a disk-shaped top board 81 and the plunger 82, which is formed protrudedly in a cylindrical shape on a lower surface of the top board 81.

At the end side of the top board 81, a plurality of claw parts 83, which is formed toward the tip direction from the top board 81, is arranged. Then, each of claw parts 83 is engaged with a rib 711, which is protrudedly formed in a ring shape along the circumferential direction at an outer peripheral part 71 of the cylindrically-shaped holding member 7. As a result, the operation member body 8 and the holding member 7 are fixed to each other (see Figs. 1 to 14). Then, when the operation member 11 is operated, for example, fingers from the forefinger to the little finger of one hand are placed on the outer peripheral part 71 of the holding member 7, and the thumb is placed on the top board 81 of the operation member body 8. Then, the operation can be surely performed.

As shown in Figs. 8 to 14, in the liquid administration device 10, the cylindrically-shaped restricting member 4 is arranged at the outer peripheral side of the inner cylinder 1, and moreover, the cylindrically-shaped cover member 6 is arranged at the outer peripheral side of the restricting member 4. These members are arranged concentrically with each other. In addition, the restricting member 4 is rotatably supported relative to the inner cylinder 1 around its center axis, and the cover member 6 is movably supported relative to the inner cylinder 1 along its center axis direction.

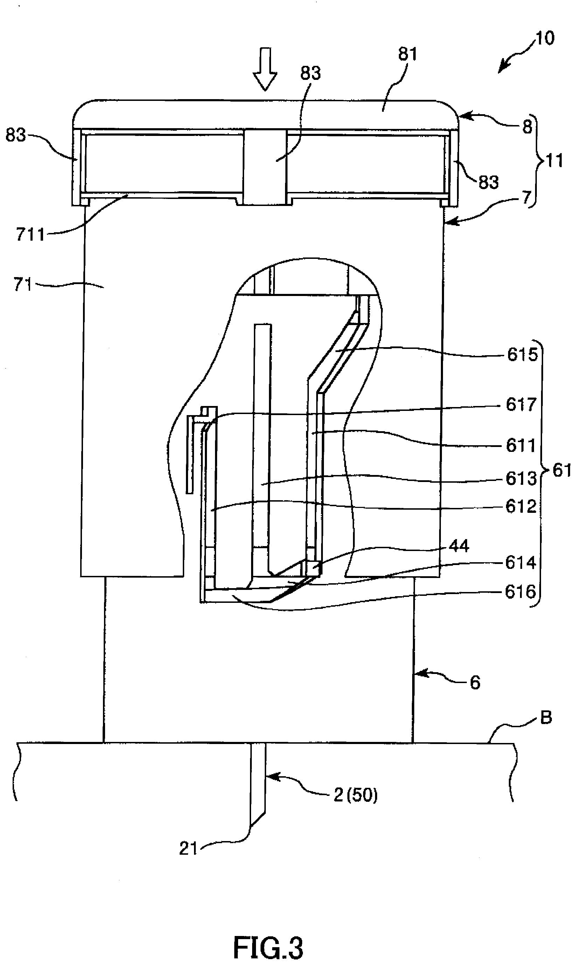

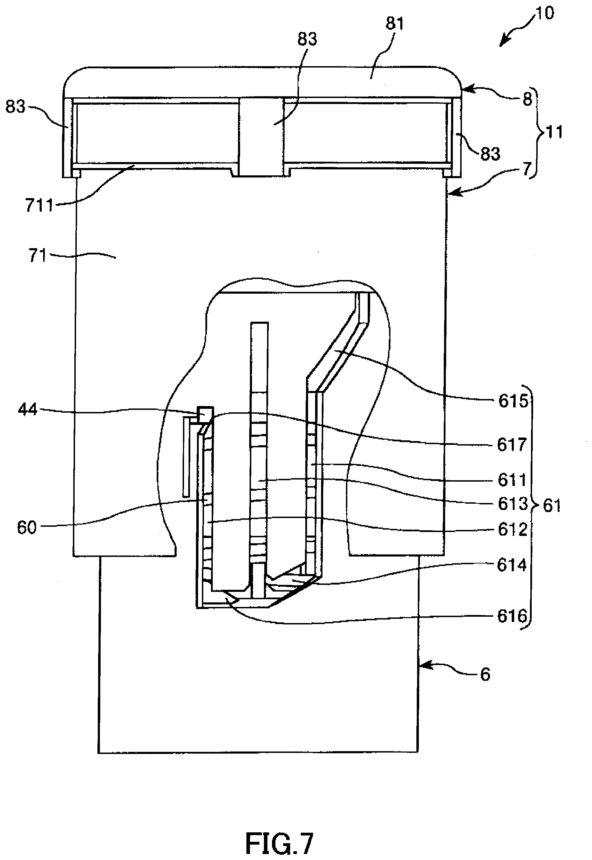

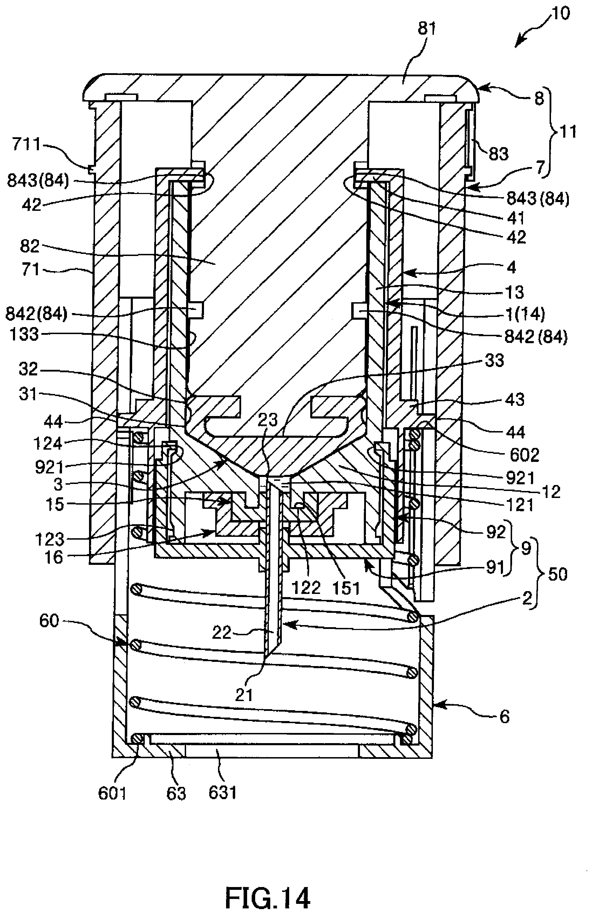

The cover member 6 can move to a first position (see Figs. 1, 2, 8, and 9), a second position (see Figs. 3 to 6, and 10 to 13), and a third position (see Figs. 7 and 14). In the first position, the cover member 6 can cover at least the tip side needle tip 21 of the double ended needle 2. As a result, erroneous puncturing by the tip side needle tip 21, or damage to the tip side needle tip 21 can be surely prevented. In the second position, shifted (retreated) from this first position to the base end direction, the tip side needle tip 21 is exposed. As a result, the living body B can be punctured with the tip side needle tip 21. In the third position, shifted from the second position toward the tip direction, at least the tip side needle tip 21 of the double ended needle 2 can be re-covered, and the movement to the second position is prevented.

This cover member 6 is biased toward the direction of moving from the second position to the first position (third position) by biasing force of the coil spring 60.

As shown in Figs. 8 to 14, the coil spring 60 is stored in the cover member 6 in a compressed state. In this coil spring 60, its tip 601 abuts on a ring-shaped tip wall part 63 of the cover member 6, and a base end 602 abuts on a rib 43 of the inner cylinder 1. Then, the coil spring 60 is compressed between them. As a result, the cover member 6 can be surely biased to the direction from the second position to the first position. By such biasing force of the coil spring 60, the tip side needle tip 21 of the puncture needle 50 can be covered with the cover member 6 before/after use of the liquid administration device 10. Thus, erroneous puncturing by the tip side needle tip 21 can be surely prevented.

A constituent material of the coil spring 60 is not particularly limited. For example, the metallic material such as stainless steel can be used.

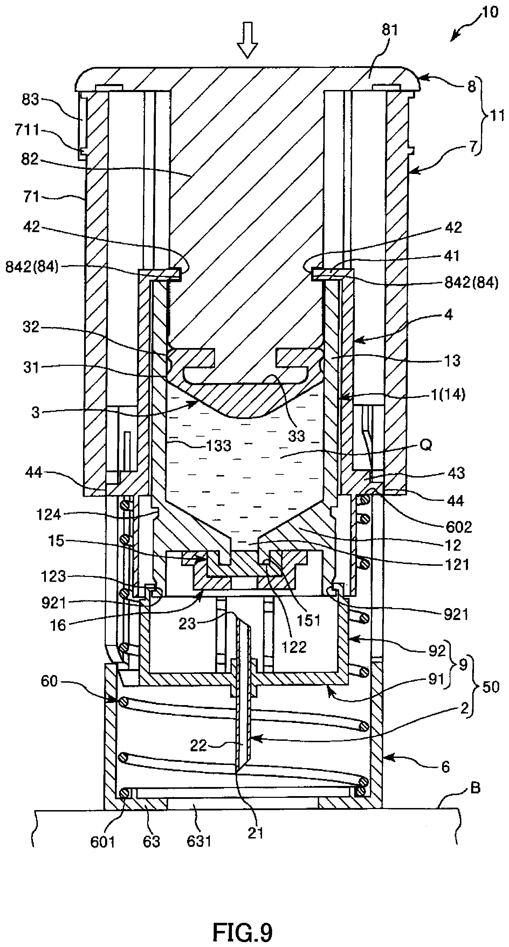

In addition, the restricting member 4 can take a pressing operation restricting state (see Figs. 8 and 9), and a pressing operation possible state (see Figs. 11 to 13) relative to the operation member 11. The pressing operation restricting state is a state, in which the pressing operation by the operation member 11 can be restricted when the cover member 6 is in the first position. The pressing operation possible state is a state, in which the pressing operation by the operation member 11 can (is allowed to) be performed when the cover member 6 is moved from the first position to the second position.

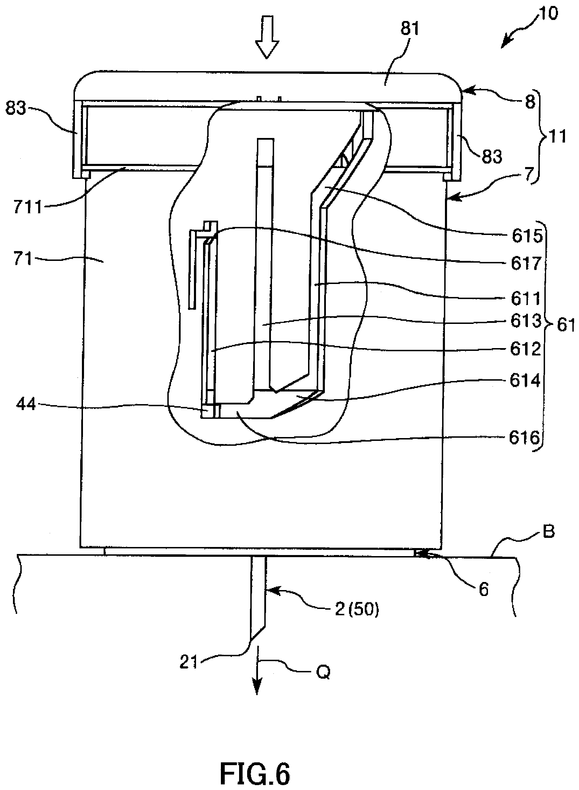

Moreover, the restricting member 4 can take a first movement possible state (see Figs. 1 to 3), a second movement possible state (see Fig. 6), and a movement restricting state (see Fig. 7) relative to the cover member 6. The first movement possible state is a state, in which the cover member 6 can (is allowed to) move from the first position to the second position. The second movement possible state is a state, in which the cover member 6 can (is allowed to) move from the second position to the third position in conjunction with completion of the pressing operation. The movement restricting state is a state, in which the cover member 6 is restricted to move back to the second position, after once the cover member 6 has been retreated to the second position and has moved to the third position.

Hereinafter, the configuration to take these states will be described.

As shown in Figs. 15 and 16, at the base end of the inner peripheral part of the restricting member 4 (one member), a rib 41 formed to be ring-shaped along its circumferential direction is protruded. Then, a plurality (four in the present embodiment) of inner protrusion parts (inner follower part) 42 is formed on this rib 41. The plurality of inner protrusion parts 42 becomes a slider part and is protruded toward inside. These inner protrusion parts 42 are arranged at equal intervals along the circumferential direction of the rib 41 (inner peripheral part of the restricting member 4).

In addition, at the outer peripheral part of the restricting member 4, the rib 43 is protruded in midway in its axis direction. The rib 43 is formed to be ring-shaped along its circumferential direction of the outer peripheral part. Then, a plurality (two in the present embodiment) of outer protrusion parts (outer follower part) 44 is formed on this rib 43. The plurality of outer protrusion parts 44 becomes an outer slider part positioned outside compared with the inner protrusion part 42, and protrudes toward outside. These outer protrusion parts 44 are arranged at equal intervals along the circumferential direction of the rib 43 (outer peripheral part of the restricting member 4).

As shown in Fig. 15, at the outer peripheral part of the plunger 82 (the other member) arranged concentrically with the restricting member 4 inside the restricting member 4, the number of inner groove parts (inner cam groove) 84 same as the inner protrusion parts 42 is formed as an inner rail part (rail part) in which the inner protrusion part 42 slides. Each of the plurality of these inner groove parts 84 is arranged, so that it corresponds to each of inner protrusion parts 42, and one corresponding inner protrusion part 42 is inserted therein.

Although the number of formation of the inner protrusion part 42 and the inner groove part 84 may be one, it is preferable to be plural. As a result, the restricting member 4 can stably rotate.

Since the configuration of each of inner groove parts 84 is the same, one inner groove part 84 will be described as a representative.

The inner groove part 84 includes an inner longitudinal groove (longitudinal direction rail) 841, an inner lateral groove (lateral direction rail) 842, and an inner inclined groove 843.

The inner longitudinal groove 841 is formed toward the same direction as an operation direction during the pressing operation by the operation member 11, that is, along the axis direction (longitudinal direction) of the plunger 82.

The inner lateral groove 842 is formed toward the direction perpendicular to the operation direction during the pressing operation, that is, along the circumferential direction of the plunger 82. This inner lateral groove 842 intersects with, that is, communicates with (continues to) the middle in the longitudinal direction of the inner longitudinal groove 841.

The inner inclined groove 843 is formed along the direction inclined to the axis direction of the plunger 82. The tip of this inner inclined groove 843 intersects with the base end of the inner longitudinal groove 841.

The inner lateral grooves 842 of the inner groove part 84 adjacent to each other communicate with each other, and four inner lateral grooves 842 of the inner groove part 84 form a ring-shaped groove as a whole.

As shown in Figs. 8 and 9, the inner protrusion part 42 is positioned in the inner lateral groove 842, when the cover member 6 is in the first position. As a result, the pressing operation restricting state, in which the pressing operation by the operation member 11 is restricted, is realized.

As shown in Figs. 11 to 13, when the cover member 6 moves from the first position to the second position against the biasing force of the coil spring 60, the inner protrusion part 42 moves from the inner lateral groove 842 to the inner longitudinal groove 841 in conjunction with the movement, and is positioned in the inner longitudinal groove 841 (including an intersection of the inner longitudinal groove 841 and the inner lateral groove 842, and an intersection of the inner longitudinal groove 841 and the inner inclined groove 843). As a result, the pressing operation possible state, in which the pressing operation by the operation member 11 can be performed, is realized. The restricting member 4 rotates, so that the inner protrusion part 42 moves from the inner lateral groove 842 to the inner longitudinal groove 841. The outer protrusion part 44 of the restricting member 4 moves in an outer groove part 61 of the cover member 6 described below, so that the rotating force is generated.

As shown in Fig. 14, the inner protrusion part 42 moves from the inner longitudinal groove 841 to the inner inclined groove 843 and proceeds in the inner inclined groove 843, until the cover member 6 returns to the first position from the second position by the biasing force of the coil spring 60. Also at this time, the restricting member 4 rotates in the same direction as described above.

As shown in Fig. 16, at the cover member 6, the number of outer groove parts (outer cam groove) 61 same as the outer protrusion parts 44 is formed as an outer rail part in which the outer protrusion part 44 slides. Each of the plurality of these outer groove parts 61 is arranged, so that it corresponds to each of outer protrusion parts 44, and one corresponding outer protrusion part 44 is inserted therein.

Although the number of formation of the outer protrusion part 44 and the outer groove part 61 may be one, it is preferable to be plural as with the present embodiment. As a result, the restricting member 4 can more stably rotate combined with the plural formation of the inner protrusion part 42 and the plural formation of the inner groove part 84.

Since the configuration of each of outer groove parts 61 is the same, one outer groove part 61 will be described as a representative.

The outer groove part 61 includes a first outer longitudinal groove (first outer longitudinal direction rail) 611, a second outer longitudinal groove (second outer longitudinal direction rail) 612, a third outer longitudinal groove 613, a first outer inclined groove (outer inclined rail) 614, a second outer inclined groove 615, an outer lateral groove (outer lateral direction rail) 616, and an outer engagement part 617. Each of first outer longitudinal groove 611 to outer lateral groove 616 is a through hole, which penetrates through the inner peripheral part into the outer peripheral part of the cover member 6, that is, through the wall part of the cylindrically-shaped cover member 6.

The first outer longitudinal groove 611 is formed toward the same direction as the operation direction during the pressing operation by the operation member 11, that is, along the axis direction of the plunger 82.

The second outer longitudinal groove 612 is formed in the position being separated relative to the first outer longitudinal groove 611 along the circumferential direction of the plunger 82, toward the opposite direction to the operation direction during the pressing operation, that is, in parallel with the first outer longitudinal groove 611.

The third outer longitudinal groove 613 is formed between the first outer longitudinal groove 611 and the second outer longitudinal groove 612, toward the opposite direction to the operation direction during the pressing operation.

The first outer inclined groove 614 is formed between the first outer longitudinal groove 611 and the third outer longitudinal groove 613, along the direction inclined to the operation direction during the pressing operation. The tip of this first outer inclined groove 614 intersects with the tip of the third outer longitudinal groove 613, and the base end of this first outer inclined groove 614 intersects with the tip of the first outer longitudinal groove 611.

The second outer inclined groove 615 is formed along the direction inclined to the operation direction during the pressing operation, and the tip thereof intersects with the based end of the first outer longitudinal groove 611.

The outer lateral groove 616 is formed between the second outer longitudinal groove 612 and the third outer longitudinal groove 613, along the direction perpendicular to the operation direction during the pressing operation. One end of this outer lateral groove 616 intersects with the base end of the second outer longitudinal groove 612, and the other end of this outer lateral groove 616 intersects with the tip of the third outer longitudinal groove 613 and the tip of the first outer inclined groove 614.

The outer engagement part 617 is the elastic piece configured by a part of the wall part of the cover member 6, which defines the second outer longitudinal groove 612. This outer engagement part 617 is arranged at the base end of the second outer longitudinal groove 612, and can be engaged with the outer protrusion part 44 from its tip side when the cover member 6 returns to the first position (see Fig. 7). In addition, the outer engagement part 617 configured by the elastic piece can be elastically deformed. Therefore, when the cover member 6 moves from the second position to the third position, the outer protrusion part 44 can go beyond the outer engagement part 617, and thus allow the movement.

As shown in Figs. 1 to 3, the outer protrusion part 44 can move in the second outer inclined groove 615, the first outer longitudinal groove 611, and the first outer inclined groove 614 in order. As a result, the first movement possible state, in which the cover member 6 can move from the first position to the second position, is realized.

In addition, the restricting member 4 can rotate when the outer protrusion part 44 moves in the first outer inclined groove 614. As described above, by this rotation, the inner protrusion part 42 can move from the inner lateral groove 842 to the inner longitudinal groove 841, and thus the pressing operation possible state, in which the pressing operation by the operation member 11 can be performed, is realized.

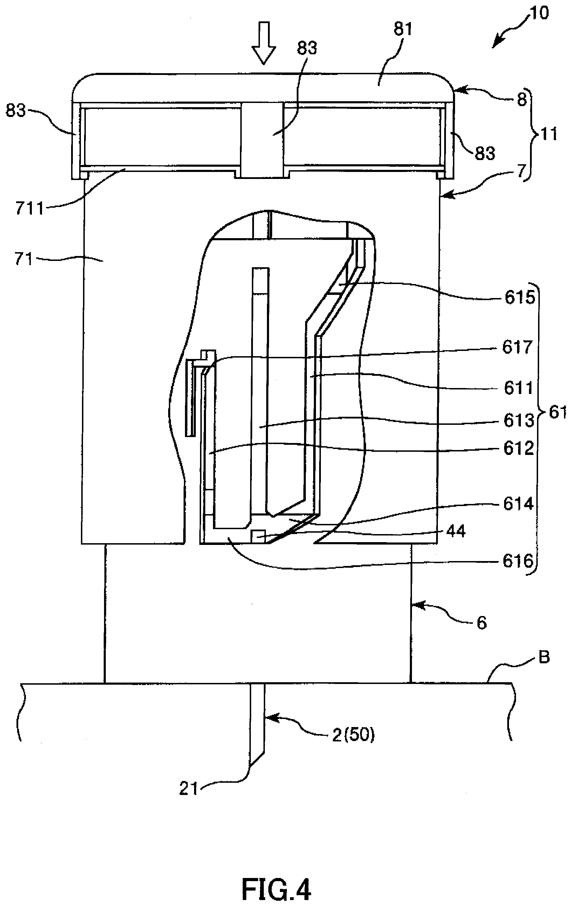

As shown in Figs. 4 to 6, the outer protrusion part 44 can move in the outer lateral groove 616, when the cover member 6 is in the second position. This movement is made by the rotation of the restricting member 4. The inner protrusion part 42 moves in the inner inclined groove 843, so that the rotating force is generated.

In addition, as shown in Fig. 6, when the outer protrusion part 44 moves to the end of the outer lateral groove 616, the outer protrusion part 44 can move subsequently in the second outer longitudinal groove 612. As a result, the second movement possible state, in which the cover member 6 can move from the second position to the third position, is realized.

As shown in Fig. 7, when the cover member 6 returns to the first position from the second position, the outer protrusion part 44 moves in the second outer longitudinal groove 612. Then, this outer protrusion part 44 is engaged with the outer engagement part 617. As a result, the movement restricting state, in which the cover member 6 is restricted to move back to the second position, is realized.

The outer groove part 61 is a through hole as described above, and the outer protrusion part 44 can be exposed. As shown in Fig. 7, in the movement restricting state, the outer protrusion part 44 and the outer engagement part 617, which is engaging with the outer protrusion part 44, are covered by the holding member 7 of the operation member 11. As a result, unintentional release of the engagement of the outer protrusion part 44 with the outer engagement part 617 can be surely prevented. Thus, the cover member 6 can surely stay in the first position. The tip side needle tip 21 is covered by this cover member 6, and erroneous puncturing and so on can be prevented.

As shown in Figs. 8 to 14, the puncture needle 50 can move relative to the inner cylinder 1 along its axis direction. Then, when the cover member 6 is in the first position, the restricting member 4 can also take a puncture needle movement restricting state, in which the movement of the puncture needle 50 (support member 9) is restricted. Next, the configuration to take this puncture needle movement restricting state will be described.

As shown in Fig. 17, at the tip of the inner peripheral part of the restricting member 4, a plurality (four in the present embodiment) of inner protrusion parts (second inner protrusion part) 45 is formed. The plurality of inner protrusion parts 45 protrudes toward inside. These inner protrusion parts 45 are arranged at equal intervals along the circumferential direction of the restricting member 4.

As shown in Fig. 18, at the outer peripheral part of the wall part 92 of the support member 9 of the puncture needle 50, the number of step parts 922 same as inner protrusion parts 45 is formed. Each of step parts 922 is formed, so that a step toward outside is generated at the outer peripheral part of the wall part 92.

When the cover member 6 is in the first position, each of inner protrusion parts 45 is engaged with each of step parts 922 from its base end side (see "inner protrusion part 45" shown in a solid line in Fig. 18). As a result, the puncture needle movement restricting state, in which the movement of the puncture needle 50 is restricted, is realized. Thus, unintentional puncturing of the double ended needle 2 to the sealing member 15 of the inner cylinder 1 is prevented before using the liquid administration device 10, and leaking of the liquid Q from the double ended needle 2 is prevented.

Then, by rotating the restricting member 4 described above from the puncture needle movement regulating state, the inner protrusion part 45 is separated (see "inner protrusion part 45" shown in a two-dot chain line in Fig. 18) from each of step parts 922, and the puncture needle movement restricting state is released. As a result, puncture needle 50 is made in a movable state to the base end side, and thus the double ended needle 2 can puncture the sealing member 15 of the inner cylinder 1.

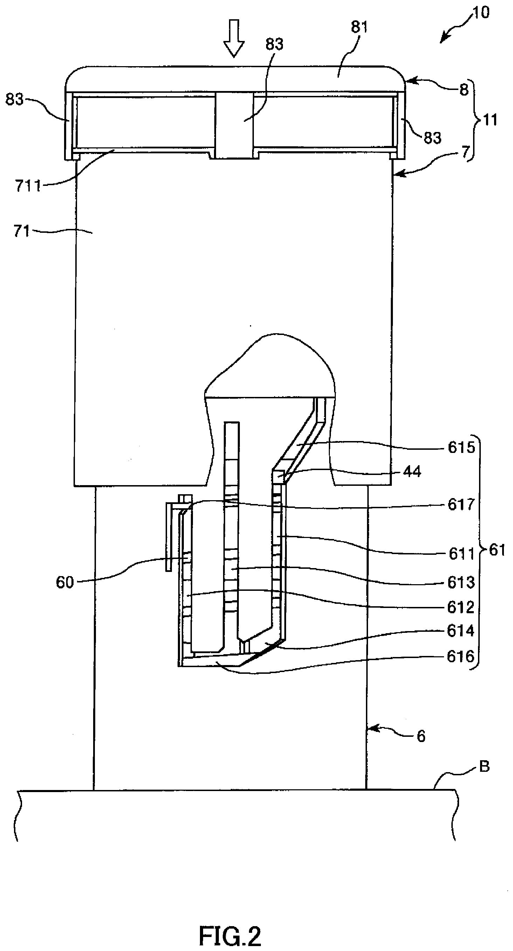

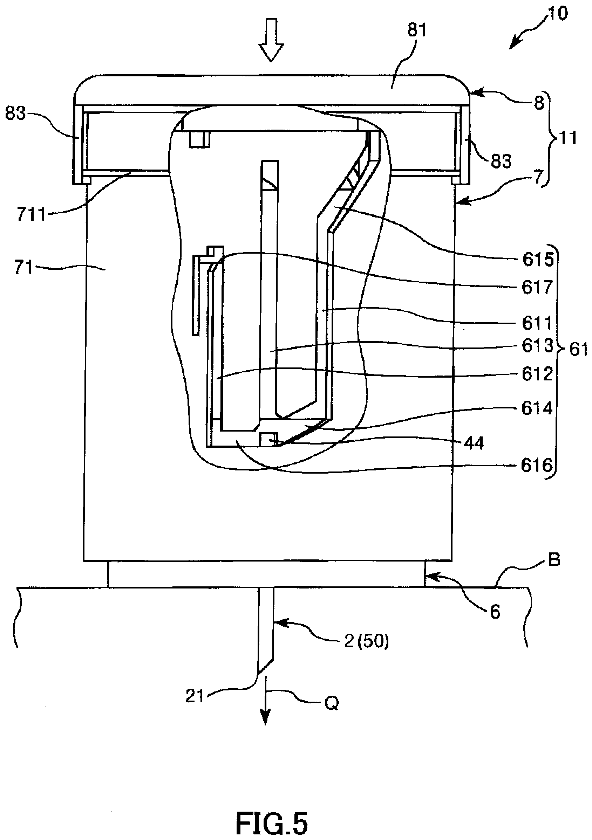

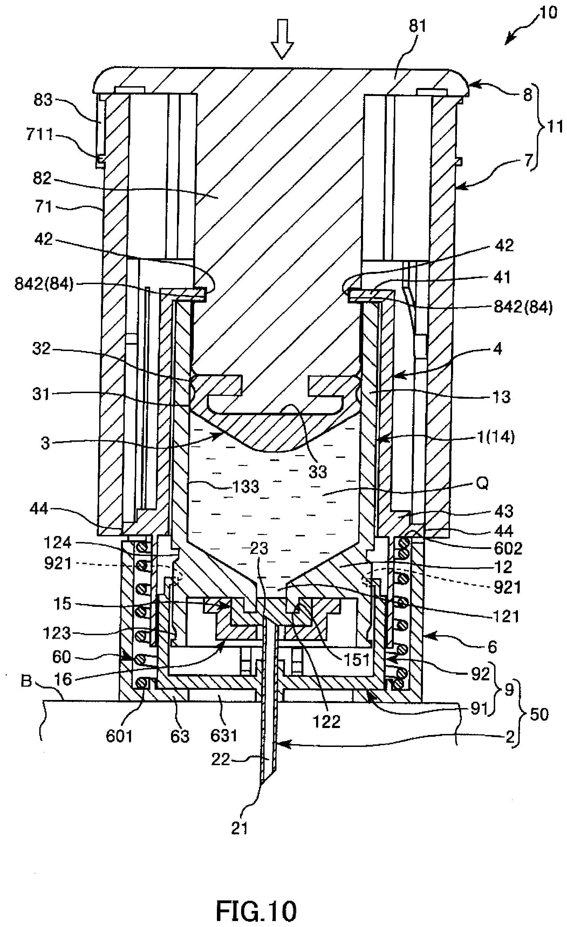

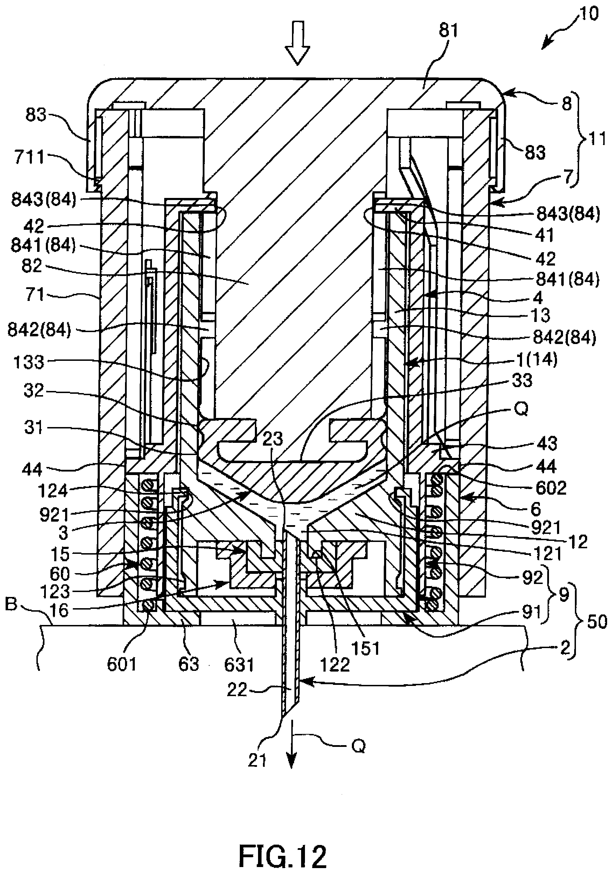

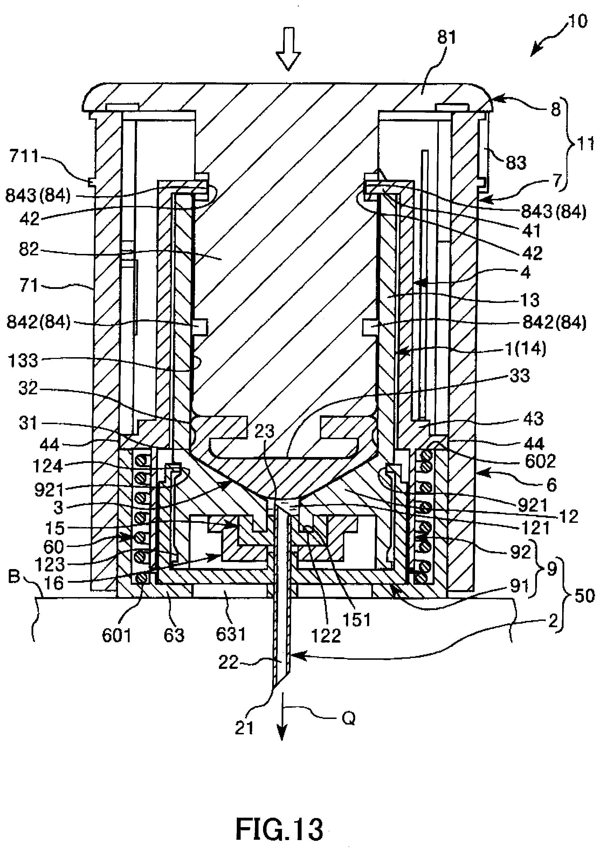

Next, the usage of the liquid administration device 10 and the operation state when in use will be described with reference to Figs. 1 to 14.
[1] The liquid administration device 10 in an unused state (initial state) as shown in Figs. 1 and 8 is prepared. In the liquid administration device 10 in this unused state, the cover member 6 is in the first position and covers the tip side needle tip 21 of the double ended needle 2. In this unused state, the tip side needle tip 21 is maintained to be covered by the cover member 6 by the biasing force of the coil spring 60. As a result, erroneous puncturing by the tip side needle tip 21 can be surely prevented.
   In addition, as shown in Fig. 8, in the puncture needle 50, the base end side needle tip 23 of the double ended needle 2 is separated from the sealing member 15 of the inner cylinder 1, and not yet piercing the sealing member 15. The state where the base end side needle tip 23 is being separated from the sealing member 15 of the inner cylinder 1 is surely maintained by the restricting member 4 in the puncture needle movement restricting state. As a result, the liquid Q can be maintained in an aseptic state until administration of the medicinal liquid Q starts.
   In addition, each of inner protrusion parts 42 is positioned in the inner lateral groove 842 of the plunger 82 of the operation member 11, and the restricting member 4 is in the pressing operation restricting state relative to the operation member 11. As a result, the pressing operation by the operation member 11 is restricted (made impossible).
   Moreover, each of outer protrusion parts 44 is positioned in the second outer inclined groove 615, and the restricting member 4 is in the movement possible state relative to the cover member 6.
[2] Next, the holding member 7 of the operation member 11 of the liquid administration device 10 in the unused state is held. As shown in Figs. 2 and 9, the tip wall part 63 of the cover member 6 is abutted on the living body B and the operation member 11 is straightly pressed toward the tip direction. As a result, the cover member 6 starts to move from the first position to the second position against the biasing force of the coil spring 60. In the moving process, each of outer protrusion parts 44 proceeds in the second outer inclined groove 615. As a result, the restricting member 4 rotates and the puncture needle movement restricting state is released. Thus, the puncture needle 50 is made in the movable state to the base end side.
[3] When the operation member 11 is pressed further toward the tip direction from the state shown in Fig. 2, each of outer protrusion parts 44 proceeds in the first outer longitudinal groove 611 (see Fig. 3), and the cover member 6 moves from a puncture prevention position to a puncture possible position. At this time, the tip side needle tip 21 of the double ended needle 2 protrudes from an opening part 631 of the tip wall part 63 of the cover member 6, and the living body B is punctured with the tip side needle tip 21 (see Figs. 3 and 10).
   In addition, with the movement of the cover member 6, the tip wall part 63 of the cover member 6 starts to press the support member 9 of the puncture needle 50 toward the base end direction (see Fig. 10).
[4] When the operation member 11 is continued to be pressed toward the tip direction from the state shown in Fig. 3, each of outer protrusion parts 44 proceeds in the first outer inclined groove 614 (see Fig. 4). As a result, the restricting member 4 rotates and the inner protrusion part 42 moves to the inner longitudinal groove 841. Thus the pressing operation possible state is realized (see Fig. 11).
   At this time, the tip wall part 63 of the cover member 6 further presses the support member 9 of the puncture needle 50 toward the base end direction. As a result, the sealing member 15 of the inner cylinder 1 can be punctured with the base end side needle tip 23 of the double ended needle 2, and thus the double ended needle 2, which has punctured the living body B, is communicated with the inner cylinder 1 (see Fig. 11).
[5] Moreover, when the operation member 11 is further continued to be pressed toward the tip direction from the state shown in Figs. 4 and 11, the inner protrusion part 42 proceeds in the inner longitudinal groove 841 and the inner inclined groove 843 in order, and moves to the limit of movement, in which the gasket 3 abuts on the bottom part 12 of the inner cylinder 1. Then, the liquid Q can be discharged from the double ended needle 2 (see Figs. 12 and 13).
   As a result, administration of the liquid Q is completed.
   The inner protrusion part 42 proceeds in the inner inclined groove 843, so that the restricting member 4 rotates. As a result, the outer protrusion part 44 proceeds in the outer lateral groove 616 up to the second outer longitudinal groove 612 (see Figs. 5 and 6).
[6] Next, as shown in Figs. 7 and 14, the liquid administration device 10 is separated from the living body B. As a result, the cover member 6 is biased toward the tip direction by the biasing force of the coil spring 60. Then, the outer protrusion part 44 proceeds in the second outer longitudinal groove 612 and returns to the first position.

At this time, the outer protrusion part 44 can be engaged with the outer engagement part 617, and thus the restricting member 4 is made in the movement restricting state relative to the cover member 6. As a result, the cover member 6 is surely restricted to move back to the second position, and erroneous puncturing by the tip side needle tip 21 is prevented.

In this manner, in the liquid administration device 10, the liquid Q is administered after the living body B is punctured. That is, the liquid administration device 10 can be used in the order "puncturing", "administration". Therefore, the pressing operation of discharging the liquid Q by the operation member 11 can be surely prevented from being performed erroneously, before the living body B is punctured.

In the liquid administration device 10, the administration can be stopped during administration of the liquid Q. In this case, when the liquid administration device 10 is separated from the living body B, the cover member 6 is biased toward the tip direction by the biasing force of the coil spring 60. As a result, the outer protrusion part 44 can proceed in the third outer longitudinal groove 613 and return to the first position.

Although the embodiment illustrated has been described above for the liquid administration device of the present invention, the present invention is not limited thereto. Each part configuring the liquid administration device can be replaced by the one having an arbitrary configuration which can exhibit the equivalent function. Besides, an arbitrary component may be added.

Although in the above-described embodiment, the puncture needle is the one having the needle tube being the double ended needle, the puncture needle is not limited thereto. The puncture needle may be the one having a needle tube without the base end side needle tip. In this case, the needle tube is preliminarily (already in the unused state) communicated with an inner cylinder.

In addition, in the above-described embodiment, the inner protrusion part is formed on the restricting member out of the operation member and the restricting member, and the inner groove part, into which the inner protrusion part is inserted, is formed on the plunger of the operation member. The invention of the present application is not limited thereto, and the inner protrusion part may be formed on the plunger of the operation member, and the inner groove part may be formed on the restricting member.

In the above-described embodiment, the outer groove part is formed on the restricting member out of the restricting member and the cover member, and the outer groove part, into which the outer protrusion part is inserted, is formed on the cover member. The invention of the present application is not limited thereto, and the outer protrusion part may be formed on the cover member, and the outer groove part may be formed on the restricting member.

In addition, in the outer groove part, the third outer longitudinal groove may be omitted.

In the above described embodiment, the explanation has been made with the restricting member having a cylindrical shape, the cylindrical shape may be short in the height direction or may be disk-shaped (doughnut-shaped) having a hole substantially in the center. Therefore, the concept of the "cylindrical shape" in the explanation of the restricting member herein includes the disk shape.

In addition, although each of the outer rail part and the inner rail part is configured by the groove in the above-described embodiment, the present invention is not limited thereto as long as the outer slider part and the inner slider part are slidable along the rail. For example, each of the outer rail part and the inner rail part may be configured by a protrusion (rib) or a step.

### Industrial Applicability

A liquid administration device of the present invention includes:
a cylindrically-shaped cylindrical body configured to be filled with liquid in its inside;
a needle tube configured to have a tip side needle tip at least at the tip out of the tip and the base end, to be provided at the tip side of the cylindrical body, and to be communicable with the cylindrical body;
a gasket configured to be capable of sliding within the cylindrical body;
an operation member configured to be coupled to the base end side of the gasket and to perform pressing operation to discharge the liquid from the needle tube by pressing the gasket toward the tip direction;
a restricting member configured to be arranged at the outer peripheral side of the cylindrical body, and to be capable of restricting the pressing operation; and
a cover member configured to be capable of moving to a first position, in which at least the tip side needle tip of the needle tube is covered, and to a second position, retreated to the base end direction from the first position, in which the tip side needle tip is exposed, wherein
an inner protrusion part is formed protrudedly on one member out of the operation member and the restricting member, and an inner groove part, into which the inner protrusion part is inserted, is formed on the other member, the inner groove part having an inner longitudinal groove formed in the same direction as an operation direction during the pressing operation, and an inner lateral groove communicating with the inner longitudinal groove and formed in the direction perpendicular to the operation direction;
when the cover member is in the first position, the inner protrusion part is positioned in the inner lateral groove and restricts the pressing operation, and
when the cover member moves from the first position to the second position, the inner protrusion part moves from the inner lateral groove to the inner longitudinal groove in conjunction with the movement, thereby the pressing operation is made possible. Therefore, it is possible to surely prevent the operation member from operating erroneously, when the operation of the operation member is desired to be restricted. In addition, since the movement of the operation member is surely restricted until the cover member is moved from a first position to a second position, increase in inner pressure of the liquid within the cylindrical body is prevented during this time.

Therefore, the liquid administration device of the present invention has industrial applicability.

### Reference Signs List

10 liquid administration device
1 inner cylinder (cylindrical body)
12 bottom part
121 opening part
122 protruded part
123 first engagement part
124 second engagement part
13 side wall
133 inner peripheral part
14 inner cylinder body
15 sealing member (sealing part)
151 recessed part
16 fixing member
2 double ended needle
21 tip side needle tip (needle tip)
22 flow path
23 base end side needle tip
3 gasket
31, 32 protruded part
33 recessed part
4 restricting member
41 rib
42 inner protrusion part (inner follower part)
43 rib
44 outer protrusion part (outer follower part)
45 inner protrusion part (second inner protrusion part)
6 cover member
61 outer groove part (outer cam groove)
611 first outer longitudinal groove (first outer longitudinal direction rail)
612 second outer longitudinal groove (second outer longitudinal direction rail)
613 third outer longitudinal groove
614 first outer inclined groove (outer inclined rail)
615 second outer inclined groove
616 outer lateral groove (outer lateral direction rail)
617 outer engagement part
63 tip wall part
631 opening part
7 holding member (holding part)
71 outer peripheral part
711 rib
8 operation member body
81 top board
82 plunger
83 claw part
84 inner groove part (inner cam groove)
841 inner longitudinal groove (longitudinal direction rail)
842 inner lateral groove (lateral direction rail)
843 inner inclined groove
9 support member
91 fixing part (support part)
92 wall part
921 engagement part
922 step part
11 operation member
50 puncture needle
60 coil spring
601 tip
602 base end
B living body
Q liquid

## Claims

1. A liquid administration device (10) comprising:
a cylindrically-shaped cylindrical body (1) configured to be filled with liquid (Q) in its inside;
a needle tube configured to be provided being communicated or communicable with the cylindrical body (1) at a tip side of the cylindrical body (1), and to have a sharp needle tip (21) at a tip;
a gasket (3) configured to be capable of sliding within the cylindrical body (1);
an operation member (11) configured to perform pressing operation to discharge the liquid from the needle tube by pressing the gasket (3) toward a tip direction;
a restricting member (4) configured to be arranged at an outer peripheral side of the cylindrical body (1), and to be capable of restricting the pressing operation; and
a cover member (6)configured to be capable of moving to a first position, in which at least the needle tip (21) of the needle tube is covered, and to a second position, retreated to a base end (602) direction from the first position, in which the needle tip (21) is exposed, **characterized in that** a slider part is provided to one member out of the operation member (11) and the restricting member (4), and a rail part, in which the slider part slides, is provided to the other member, the rail part having a longitudinal direction rail (841) formed in the same direction as an operation direction during the pressing operation, and a lateral direction rail (842) continued to the longitudinal direction rail (841) and formed in the direction perpendicular to the operation direction;
when the cover member (6) is in the first position, the slider part is positioned in the lateral direction rail (842) and makes the pressing operation impossible, and
when the cover member (6) moves from the first position to the second position, the slider part moves from the lateral direction rail (842) to the longitudinal direction rail (841) in conjunction with the movement, thereby the pressing operation is made possible.

2. The liquid administration device (10) according to claim 1, wherein
the restricting member (4) is cylindrically-shaped;
the operation member (11) has a column-shaped plunger (82) arranged concentrically with the restricting member (4) inside the restricting member; and
the slider part is provided in an inner peripheral part of the restricting member (4) along its circumferential direction, and the rail part is arranged so as to correspond to each of the slider parts in an outer peripheral part of the plunger (82).

3. The liquid administration device (10) according to claim 1 or 2, wherein
the restricting member (4) is cylindrically-shaped;
the cover member (6) is arranged concentrically with the restricting member (4) outside the restricting member (4) and is cylindrically-shaped;
an outer slider part, positioned outside compared with the slider part, is provided in one of the outer peripheral part of the restricting member (4) and the inner peripheral part of the cover member, and an outer rail part, in which the outer slider part slides, is provided in the other, the outer rail part having an outer inclined rail (614) formed in the direction inclined relative to the operation direction; and
in the process in which the cover member (6) moves from the first position to the second position, the outer slider part slides in the outer inclined rail (614), such that the restricting member (4) rotates around its axis and the slider part slides from the lateral direction rail (842) to the longitudinal direction rail (841), thereby the pressing operation is made possible.

4. The liquid administration device (10) according to any one of claims 1 to 3, wherein
the cover member (6) can further move to a third position, in which the needle tip (21) is covered and the movement to the second position is prevented; and
the restricting member (4) is configured to allow the movement of the cover member (6) from the second position to the third position in conjunction with completion of the pressing operation.

5. The liquid administration device (10) according to claim 4, wherein
the outer rail part has a first outer longitudinal direction rail (841) continued to the base end of the outer inclined rail (614) and formed in the same direction as the operation direction during the pressing operation, an outer lateral direction rail (842) continued to a tip of the outer inclined rail (614) and formed in the direction perpendicular to the operation direction, and a second outer longitudinal direction rail (841) continued to the outer lateral direction rail (842) and formed in the opposite direction to the operation direction during the pressing operation; and
the outer slider part slides in the first outer longitudinal direction rail (841) and the outer inclined rail (614) in order in the process in which the cover member (6) moves from the first position to the second position, the outer slider part slides in the outer lateral direction rail (842) when the cover member (6) is in the second position, and the outer slider part moves in the second outer longitudinal direction rail (841) when the cover member (6) moves from the second position to the third position.

6. The liquid administration device (10) according to claim 5, wherein
the outer rail part has an engagement part, which restricts the cover member (6) to move back to the second position by engaging with the outer slider part from its tip side when the cover member (6) moves to the third position.

7. The liquid administration device (10) according to claim 6, wherein
the engagement part is deformed elastically and allows movement of the cover member (6) from the second position to the third position.

## Patentansprüche

1. Flüssigkeit verabreichende Vorrichtung (10), die Folgendes umfasst:
einen zylindrisch geformten zylindrischen Körper (1), der dafür konfiguriert ist, in seinem Inneren mit Flüssigkeit (Q) gefüllt zu werden,
eine Nadelröhre, die dafür konfiguriert ist, mit dem zylindrischen Körper (1) an einer Spitzenseite des zylindrischen Körpers (1) verbunden oder verbindbar zu sein und eine scharfe Nadelspitze (21) an einer Spitze zu haben,
eine Dichtung (3), die dafür konfiguriert ist, zum Gleiten innerhalb des zylindrischen Körpers (1) in der Lage zu sein,
ein Betätigungselement (11), das dafür konfiguriert ist, durch das Pressen der Dichtung (3) zu einer Spitzenrichtung hin eine Pressbetätigung durchzuführen, um die Flüssigkeit aus der Nadelröhre abzugeben,
ein Beschränkungselement (4), das dafür konfiguriert ist, an einer Außenumfangsseite des zylindrischen Körpers (1) angeordnet zu werden und dazu in der Lage zu sein, die Pressbetätigung zu beschränken, und
ein Abdeckelement (6), das dafür konfiguriert ist, in der Lage zu sein zum Bewegen zu einer ersten Position, in der wenigstens die Nadelspitze (21) der Nadelröhre abgedeckt ist, und zu einer zweiten Position, zurückgezogen aus der ersten Position zu einer Richtung eines Basisendes (602), in der die Nadelspitze (21) freigelegt ist, **dadurch gekennzeichnet, dass**
ein Gleitstückteil an dem einen Element von dem Betätigungselement (11) und dem Beschränkungselement (4) bereitgestellt wird und ein Schienenteil, in dem das Gleitstückteil gleitet, an dem anderen Element bereitgestellt wird, wobei das Schienenteil eine Längsrichtungsschiene (841), die in der gleichen Richtung wie eine Betätigungsrichtung während der Pressbetätigung geformt ist, und eine Seitenrichtungsschiene (842), die sich an der Längsrichtungsschiene (841) fortsetzt und in der Richtung, senkrecht zu der Betätigungsrichtung, geformt ist, hat,
wenn sich das Abdeckelement (6) in der ersten Position befindet, das Gleitstückteil in der Seitenrichtungsschiene (842) angeordnet ist und die Pressbetätigung unmöglich macht und,
wenn sich das Abdeckelement (6) von der ersten Position zu der zweiten Position bewegt, sich das Gleitstückteil in Verbindung mit der Bewegung von der Seitenrichtungsschiene (842) zu der Längsrichtungsschiene (841) bewegt, wodurch die Pressbetätigung möglich gemacht wird.

2. Flüssigkeit verabreichende Vorrichtung (10) nach Anspruch 1, wobei
das Beschränkungselement (4) zylindrisch geformt ist, das Betätigungselement (11) einen säulenförmigen Druckkolben (82) hat, der konzentrisch mit dem Beschränkungselement (4) innerhalb des Beschränkungselements angeordnet ist, und
das Gleitstückteil in einem Innenumfangsteil des Beschränkungselements (4) entlang seiner Umfangsrichtung angeordnet ist und das Schienenteil so angeordnet ist, dass es jedem der Gleitstückteile in einem Außenumfangsteil des Druckkolbens (82) entspricht.

3. Flüssigkeit verabreichende Vorrichtung (10) nach Anspruch 1 oder 2, wobei
das Beschränkungselement (4) zylindrisch geformt ist, das Abdeckelement (6) konzentrisch mit dem Beschränkungselement (4) außerhalb des Beschränkungselements (4) angeordnet ist und zylindrisch geformt ist,
ein äußeres Gleitstückteil, das, verglichen mit dem Gleitstückteil, außerhalb angeordnet ist, in dem einen von dem Außenumfangsteil des Beschränkungselements (4) und dem Innenumfangsteil des Abdeckelements bereitgestellt wird und ein äußeres Schienenteil, in dem das äußere Gleitstückteil gleitet, in dem anderen bereitgestellt wird, wobei das äußere Schienenteil eine äußere geneigte Schiene (614) hat, die in der im Verhältnis zu der Betätigungsrichtung geneigten Richtung geformt ist, und
in dem Vorgang, in dem sich das Abdeckelement (6) von der ersten Position zu der zweiten Position bewegt, das Gleitstückteil derart in der äußeren geneigten Schiene (614) gleitet, dass sich das Beschränkungselement (4) um seine Achse dreht und das Gleitstückteil von der Seitenrichtungsschiene (842) zu der Längsrichtungsschiene (841) gleitet, wodurch die Pressbetätigung möglich gemacht wird.

4. Flüssigkeit verabreichende Vorrichtung (10) nach einem der Ansprüche 1 bis 3, wobei
sich das Abdeckelement (6) ferner zu einer dritten Position bewegen kann, in der die Nadelspitze (21) abgedeckt ist und die Bewegung zu der zweiten Position verhindert wird, und
das Beschränkungselement (4) dafür konfiguriert ist, die Bewegung des Abdeckelements (6) von der zweiten Position zu der dritten Position in Verbindung mit dem Abschließen der Pressbetätigung zu ermöglichen.

5. Flüssigkeit verabreichende Vorrichtung (10) nach Anspruch 4, wobei
das äußere Schienenteil eine erste äußere Längsrichtungsschiene (841), die sich an dem Basisende der äußeren geneigten Schiene (614) fortsetzt und in der gleichen Richtung wie die Betätigungsrichtung während der Pressbetätigung geformt ist, eine äußere Seitenrichtungsschiene (842), die sich an einer Spitze der äußeren geneigten Schiene (614) fortsetzt und in der Richtung, senkrecht zu der Betätigungsrichtung, geformt ist, und eine zweite äußere Längsrichtungsschiene (841), die sich an der äußeren Seitenrichtungsschiene (842) fortsetzt und in der entgegengesetzten Richtung zu der Betätigungsrichtung während der Pressbetätigung geformt ist, hat und
das äußere Gleitstückteil in der ersten äußeren Längsrichtungsschiene (841) und der äußeren geneigten Schiene (614) gleitet, damit in dem Vorgang, in dem sich das Abdeckelement (6) von der ersten Position zu der zweiten Position bewegt, das äußere Gleitstückteil in der Seitenrichtungsschiene (842) gleitet, wenn sich das Abdeckelement (6) in der zweiten Position befindet, und sich das Gleitstückteil in der zweiten äußeren Längsrichtungsschiene (841) bewegt, wenn sich das Abdeckelement (6) von der zweiten Position zu der dritten Position bewegt.

6. Flüssigkeit verabreichende Vorrichtung (10) nach Anspruch 5, wobei
das äußere Schienenteil ein Eingriffsteil hat, welches das Abdeckelement (6) beschränkt, sich zurück zu der zweiten Position zu bewegen, durch das Ineinandergreifen mit dem äußeren Gleitstückteil, wenn sich das Abdeckelement (6) zu der dritten Position bewegt.

7. Flüssigkeit verabreichende Vorrichtung (10) nach Anspruch 6, wobei
das Eingriffsteil elastisch verformt wird und eine Bewegung des Abdeckelements (6) von der zweiten Position zu der dritten Position ermöglicht.

## Revendications

1. Dispositif (10) d'administration de liquide comprenant :
un corps cylindrique (1) formé de manière cylindrique, conçu pour être rempli de liquide (Q) dans sa partie intérieure ;
un tube d'aiguille conçu pour être placé en communication ou pour pouvoir communiquer avec le corps cylindrique (1) au niveau d'un côté d'extrémité du corps cylindrique (1), et pour avoir une pointe d'aiguille pointue (21) au niveau d'une extrémité ;
un joint d'étanchéité (3) conçu pour pouvoir coulisser à l'intérieur du corps cylindrique (1) ;
un élément d'actionnement (11) conçu pour effectuer une opération de pression afin d'évacuer le liquide du tube d'aiguille en appliquant une pression sur le joint d'étanchéité (3) en direction d'une extrémité ;
un élément de limitation (4) conçu pour être disposé au niveau d'un côté périphérique extérieur du corps cylindrique (1), et pour pouvoir limiter l'opération de pression ; et
un élément de protection (6) conçu pour pouvoir se déplacer dans une première position, dans laquelle au moins la pointe d'aiguille (21) du tube d'aiguille est couverte, et dans une seconde position, déplacée en direction d'une extrémité de base (602) par rapport à la première position, dans laquelle la pointe d'aiguille (21) est exposée, **caractérisé en ce que**
une partie de coulissement est prévue sur un élément parmi l'élément d'actionnement (11) et l'élément de limitation (4), et une partie rail, dans laquelle coulisse la partie de coulissement, est prévue sur l'autre élément, la partie rail comportant un rail de direction longitudinale (841) formé dans la même direction qu'une direction de fonctionnement lors de l'opération de pression, et un rail de direction latérale (842) qui s'étend jusqu'au rail de direction longitudinale (841) et est formé dans la direction perpendiculaire à la direction de fonctionnement, ;
lorsque l'élément de protection (6) est dans la première position, la partie de coulissement est placée dans le rail de direction latérale (842) et rend l'opération de pression impossible, et lorsque l'élément de protection (6) se déplace de la première position à la seconde position, la partie de coulissement passe du rail de direction latérale (842) au rail de direction longitudinale (841) en même que le déplacement, ce qui rend ainsi l'opération de pression possible.

2. Dispositif (10) d'administration de liquide selon la revendication 1, dans lequel :
l'élément de limitation (4) est de forme cylindrique ;
l'élément d'actionnement (11) comporte un piston en forme de colonne (82) disposé de façon concentrique à l'élément de limitation (4) à l'intérieur de l'élément de limitation ; et
la partie de coulissement est prévue dans une partie périphérique intérieure de l'élément de limitation (4) le long de sa direction circonférentielle, et la partie rail est placée de manière à correspondre à chacune des parties de coulissement dans une partie périphérique extérieure du piston (82).

3. Dispositif (10) d'administration de liquide selon la revendication 1 ou 2, dans lequel :
l'élément de limitation (4) est de forme cylindrique ;
l'élément de protection (6) est disposé de façon concentrique à l'élément de limitation (4) à l'extérieur de l'élément de limitation (4) et est de forme cylindrique ;
une partie de coulissement extérieure, placée à l'extérieur par rapport à la partie de coulissement, est prévue dans l'une de la partie périphérique extérieure de l'élément de limitation (4) et de la partie périphérique intérieure de l'élément de protection, et une partie de rail extérieur, dans laquelle coulisse la partie de coulissement extérieure, est prévue dans l'autre, la partie de rail extérieur comportant un rail incliné extérieur (614) formé dans la direction inclinée par rapport à la direction de fonctionnement ; et
lors de l'opération dans laquelle l'élément de protection (6) passe de la première position à la seconde position, la partie de coulissement extérieure coulisse dans le rail incliné extérieur (614), de telle sorte que l'élément de limitation (4) tourne autour de son axe et la partie de coulissement coulisse du rail de direction latérale (842) avers le rail de direction longitudinale (841), ce qui permet ainsi l'opération de pression.

4. Dispositif (10) d'administration de liquide selon l'une quelconque des revendications 1 à 3, dans lequel :
l'élément de protection (6) peut en outre se déplacer dans une troisième position, dans laquelle la pointe d'aiguille (21) est couverte et le déplacement vers la seconde position est empêché ; et
l'élément de limitation (4) est adapté pour permettre le déplacement de l'élément de protection (6) de la seconde position à la troisième position en relation avec la fin de l'opération de pression.

5. Dispositif (10) d'administration de liquide selon la revendication 4, dans lequel :
la partie de rail extérieur comprend un premier rail extérieur de direction longitudinale (841) qui s'étend jusqu'à l'extrémité de base du rail incliné extérieur (614) et est formé dans la même direction que la direction de fonctionnement lors de l'opération de pression, un rail extérieur de direction latérale (842) qui s'étend jusqu'à une extrémité du rail incliné extérieur (614) et est formé dans la direction perpendiculaire à la direction de fonctionnement, et un second rail extérieur de direction longitudinale (841) qui s'étend jusqu'au rail extérieur de direction latérale (842) et est formé dans la direction opposée à la direction de fonctionnement lors de l'opération de pression ; et
la partie de coulissement extérieure coulisse dans le premier rail extérieur de direction longitudinale (841) et dans le rail incliné extérieur (614) afin que lors de l'opération dans laquelle l'élément de protection (6) passe de la première position à la seconde position, la partie de coulissement extérieure coulisse dans le rail extérieur de direction latérale (842) lorsque l'élément de protection (6) se trouve dans la seconde position, et la partie de coulissement extérieure se déplace dans le second rail extérieur de direction longitudinale (841) lorsque l'élément de protection (6) passe de la seconde position à la troisième position.

6. Dispositif (10) d'administration de liquide selon la revendication 5, dans lequel :
la partie de rail extérieur comporte une partie d'engagement, qui empêche l'élément de protection (6) de revenir dans la seconde position en venant en contact avec la partie de coulissement extérieure depuis son côté d'extrémité lorsque l'élément de protection (6) se déplace dans la troisième position.

7. Dispositif (10) d'administration de liquide selon la revendication 6, dans lequel :
la partie d'engagement est déformée élastiquement et permet le déplacement de l'élément de protection (6) de la seconde position à la troisième position.
